Europäisches Patentamt

(19) European Patent Office

Office européen des brevets

(11) Veröffentlichungsnummer: **0 032 204**
**B1**

(12) # EUROPÄISCHE PATENTSCHRIFT

(45) Veröffentlichungstag der Patentschrift:
31.07.85

(51) Int. Cl.⁴: **G 01 N 33/543**, G 01 N 33/573

(21) Anmeldenummer: **80107737.1**

(22) Anmeldetag: **09.12.80**

(54) **Verfahren und Mittel zur immunologischen Bestimmung von Enzymen.**

(30) Priorität: **27.12.79 DE 2952478**

(43) Veröffentlichungstag der Anmeldung:
**22.07.81 Patentblatt 81/29**

(45) Bekanntmachung des Hinweises auf die Patenterteilung:
**31.07.85 Patentblatt 85/31**

(84) Benannte Vertragsstaaten:
**AT BE CH DE FR GB IT LI NL SE**

(56) Entgegenhaltungen:
WO - A - 79/00475
FR - A - 2 192 144
FR - A - 2 209 459
FR - A - 2 253 552
US - A - 3 932 221

THE JOURNAL OF BIOLOGICAL CHEMISTRY, Band 234, Nr. 6, 1959, Seiten 1433-1436 MAX SCHLAMOWITZ et al.: "Tissue sources of human serum alkaline phosphatase, as determined by immunochemical procedures"
CLINICAL CHEMISTRY, Band 25, Nr. 5, Mai 1979, Seiten 729-734 Easton, Pennsylvania, U.S.A. M. USATEGUI-GOMEZ et al.: "Immunochemical determination of the heart isoenzyme of lactate dehydrogenase (LDH1) in human serum"

(73) Patentinhaber: **Merck Patent Gesellschaft mit beschränkter Haftung, Frankfurter Strasse 250, D-6100 Darmstadt (DE)**

(72) Erfinder: **Neumann, Siegfried, Dr., August-Metz-Weg 15, D-6100 Darmstadt (DE)**
Erfinder: **Hennrich, Norbert, Dr., Haydnweg 14, D-6100 Darmstadt (DE)**
Erfinder: **Orth, Hans-Dieter, Dr., Raiffeisenstrasse 3, D-6101 Bickenbach (DE)**
Erfinder: **Pfleiderer, Gerhard, Prof. Dr., Sombartstrasse 18, D-7000 Stuttgart 80 (DE)**
Erfinder: **Jockers-Wretou, Evangelista, Dr., Roma Strasse 27, Alimos Athen (GR)**
Erfinder: **Pauly, Hans, Dr., Finkenstrasse 1, D-3563 Dautphetal 2 (DE)**

(56) Entgegenhaltungen: (Fortsetzung)
W. Vogt, Enzymimmunoassay, Stuttgart 1978, Seiten 42-45, 52,53

**Beschreibung**

Die Erfindung betrifft Verfahren und Mittel zur immunologischen Bestimmung von Enzymen, insbesondere zur quantitativen immunologischen Bestimmung von Isoenzymen der sauren bzw. alkalischen Phosphatase in Körperflüssigkeiten.

Die quantitative Bestimmung von Isoenzymen im Blut hat in den letzten Jahren für die Differentialdiagnose verschiedener innerer Erkrankungen des Menschens erheblich an Bedeutung gewonnen. Im Blut von Tumorpatienten treten z. B. bestimmte Isoenzyme der alkalischen Phosphatase, der sauren Phosphatase, der Lactatdehydrogenase oder der Galactosyl-transferase in signifikant erhöhter Konzentration auf. Ihre quantitative Bestimmung gibt der klinischen Chemie wichtige Meßgrößen für die frühe Erkennung von Tumoren, die Beobachtung des Verlaufs und die Kontrolle des Therapieerfolgs in die Hand.

Es ist bekannt, daß die saure Phosphatase (Orthophosphoric monoester phosphohydrolase, EC 3.1.3.2) des Menschen in mehreren Isoenzymen vorkommt. Das Prostata-Isoenzym der sauren Phosphatase hat deutlich erhöhte Konzentrationen im Blut vieler Patienten mit Prostata-Tumor. Der Nachweis dieses Isoenzyms hat deshalb für die Erkennung früher Stadien der Krankheit und ihre Therapie große Bedeutung.

Auch für die alkalische Phosphatase (Orthophosphoric monoester phosphohydrolase, EC 3.1.3.1) des Menschen wurden mehrere biochemisch unterscheidbare Isoenzyme beschrieben. In der klinischen Chemie ist die quantitative Bestimmung der Isoenzyme aus Leber und Knochen bzw. aus Placenta im Blut wichtig. Für die Diagnostik von Nierenerkrankungen erscheint neuerdings auch die Identifizierung und quantitative Bestimmung der sogenannten cytoplasmatischen Dünndarm-AP aus der Niere im Harn interessant. Das Placenta-Isoenzym der alkalischen Phosphatase tritt im Blut schwangerer Frauen auf. Ein biochemisch und immunologisch identisch reagierendes Enzym, das sogenannte Regan-Isoenzym der alkalischen Phosphatase, wurde in erhöhten Konzentrationen im Blut von Patienten mit verschiedenen Tumoren und besonders gehäuft bei Tumoren der Testes bzw. der Ovarien gefunden.

Plasma oder Serum enthält meist Gemische von Isoenzymen der genannten Phosphatasen. Eine einfache Messung der Gesamtaktivität der sauren bzw. alkalischen Phosphatase liefert deshalb keine klaren Aufschlüsse über das Vorkommen und die Aktivität der spezifisch gesuchten Isoenzymkomponenten im Blut. Häufig stellen die gesuchten Isoenzyme auch nur einen kleinen Anteil an der gesamten Aktivität im Blut dar, so daß ihre Aktivitätsbestimmung ohne vorherige Konzentrierung der Probe nur sehr ungenau ist. Bei vielen Aktivitätsbestimmungen der Isoenzyme der alkalischen Phosphatase bzw. des Prostata-Isoenzyms der sauren Phosphatase werden für diese Bestimmungen Aliquots der biologischen Proben eingesetzt, ohne daß vorher das Enzym von der Probeflüssigkeit abgetrennt wird. Dabei bleibt unberücksichtigt, daß die Proben Störfaktoren wie z. B. im Test interferierende Enzyme oder gerinnungshemmende Zusätze oder Metaboliten von Pharmaka enthalten können (Clin. Chem. 21, 1 D-432 D (1975)).

Die herkömmlichen Methoden der Bestimmung des Prostata-Isoenzyms der sauren Phosphatase verwenden als Meßgröße die durch Enzymeinwirkung aus einem chromogenen Substrat freigesetzte Menge eines Chromophors. In neueren Methoden werden spezifische Antikörper gegen saure Phosphatase zur Erkennung und zum quantitativen Nachweis verwendet. Diese immunologischen Methoden, wie die Elektroimmunodiffusion, die Gegenstrom-Immunoelektrophorese, der Radioimmunoassay und der Fluoreszenzimmunoassay haben jedoch erhebliche Nachteile. So ist z. B. die Elektroimmunodiffusion langwierig und für Routinebestimmungen ungeeignet, die Gegenstrom-Immunoelektrophorese liefert nur semiquantitative Ergebnisse. Der Radioimmunoassay ist zeitaufwendig und nachteilig wegen der Verwendung radioaktiver Substanzen. Besondere Nachteile sind die Durchführung in einem speziellen Laboratorium, die Strahlungsgefahr und die geringe Haltbarkeit der radioaktiv markierten Reagenzien. Der Fluoreszenzimmunoassay ist technisch relativ kompliziert und fehleranfällig wegen der erforderlichen Pipettierung eines partikulären Reagenzes; dies bringt Dosierungsprobleme mit sich. Außerdem ist er nur teilweise mechanisierbar und benötigt ein spezielles Meßgerät.

Die Bestimmung der alkalischen Phosphatase aus Placenta im Serum von Schwangeren und von Tumorpatienten (Regan-Isoenzym) erfolgte bisher vor allem durch die Messung der Enzymaktivität nach Hitzebehandlung der Probe oder in Gegenwart von Inhibitoren oder durch Differenzierung der Isoenzym-Verteilung durch Kombination von Hitzevorbehandlung der Probe und Messung in Gegenwart von Inhibitoren. Diese Methoden haben den Nachteil, daß sie zu unempfindlich und nicht genügend selektiv sind. Auch die mit elektrophoretischen Methoden auf verschiedenen Trägern erzielten Ergebnisse sind nicht befriedigend, weil sie keine quantitative Aussage erlauben, teilweise Artefakte liefern und nicht als Routinebestimmungen einsetzbar sind. In neueren Methoden werden spezifische Antikörper gegen die Isoenzyme der alkalischen Phosphatasen zur Erkennung und zum quantitativen Nachweis eingesetzt. Die Präzipitation der AP-Isoenzyme durch spezifische Antikörper in freier oder immobilisierter Form ist unempfindlich und störanfällig. In anderen Techniken wird die AP an kovalent vernetztes Antiserum gebunden und in gebundener Form gemessen. Nachteile dieser Technik sind die aufwendige Herstellung des Reagenzes, seine schlechte Dosierbarkeit und die umständliche Abtrennung durch Zentrifugation. Andere immunologische Methoden wie die einfache radiale Immunodiffusion, die Elektroimmunodiffusion und Radioimmunoassays haben die gleichen Nachteile, wie sie für die Bestimmung der sauren Phosphatase bereits beschrieben wurden.

Aus der PCT-Anmeldung Nr. WO 79/00475 ist z. B. ein Verfahren zur immunologischen Bestimmung des Prostata-Isoenzyms der sauren Phosphatase auf der Basis eines Festphasen-Fluoreszenzimmunoassays bekannt. Dabei werden zunächst spezifische Antikörper gegen dieses Isoenzym kovalent an ein mit einem Cyanhalogenid aktiviertes Sepharosegel gebunden. Anschließend müssen die verbliebenen aktiven Gruppen am Sepharosegel blockiert und das überschüssige Blockierungsreagenz muß entfernt werden, wobei zusätzliche Waschschritte erforderlich sind. Die Bestimmung des an den Antikörper gebundenen Prostata-Isoenzyms der sauren Phosphatase erfolgt dann über die Fluoreszenzmessung eines Produkts, das bei der Spaltung des Substrats entsteht.

Auch dieses Verfahren hat, abgesehen von den bereits geschilderten Nachteilen im Zusammenhang mit der Auswertung über Fluoreszenzmessungen, den wesentlichen Nachteil, daß die Antikörper durch aufwendige Methoden zunächst kovalent an einen Träger gebunden werden müssen. Durch die Verwendung eines Trägers in Partikelform entsteht außerdem das Problem der schlechten Dosierbarkeit einer Partikelsuspension sowie von Zentrifugationsschritten bei der Durchführung der Bestimmung.

Es sind auch Verfahren bekannt, bei denen eine immunologische Komponente nicht nur kovalent, sondern auch adsorptiv an einen wasserunlöslichen Träger gebunden werden kann (DE-A-2 901 391, US-A-4 016 043). In diesen Fällen besteht jedoch der Bindungspartner aus einem kovalenten Kupplungsprodukt aus Antigen oder Antikörper und einem Enzym, so daß auch in diesen Fällen zunächst eine kovalente Bindung geknüpft werden muß.

Der vorliegenden Erfindung liegt die Aufgabe zugrunde, Verfahren und Mittel zur quantitativen immunologischen Bestimmung von Enzymen zur Verfügung zu stellen, mit denen die geschilderten Nachteile vermieden werden können und bei mindestens gleicher Empfindlichkeit in erheblich kürzerer Zeit die Analysenergebnisse vorliegen.

Gegenstand der Erfindung ist ein Verfahren zur quantitativen immunologischen Bestimmung von Enzymen in Flüssigkeiten, das dadurch gekennzeichnet ist, daß man die an einem wasserunlöslichen Träger adsorbierten Antikörper gegen das zu bestimmende Enzym mit der dieses Enzym enthaltenden Probelösung inkubiert, wobei das Enzym im Komplex mit den Antikörpern einen reproduzierbaren Anteil seiner Aktivität behält, die Probelösung entfernt, den mit Enzym beladenen Antikörper-Träger wäscht und die Aktivität des an die Antikörper gebundenen Enzyms bestimmt.

Gegenstand von Weiterbildungen der Erfindung sind insbesondere Verfahren zur quantitativen immunologischen Bestimmung von Isoenzymen der sauren Phosphatase, vor allem des Prostata-Isoenzyms der sauren Phosphatase und der alkalischen Phosphatase in Körperflüssigkeiten, die dadurch gekennzeichnet sind, daß man die an einem wasserunlöslichen Träger adsorbierten Antikörper gegen das zu bestimmende Phosphatase-Isoenzym mit der dieses Isoenzym enthaltenen Probelösung inkubiert, wobei das Isoenzym im Komplex mit den Antikörpern einen reproduzierbaren Anteil seiner Aktivität behält, die Probelösung entfernt, den mit Isoenzym beladenen Antikörper-Träger wäscht und die Aktivität des an die Antikörper gebundenen Isoenzyms bestimmt.

Ferner betrifft die Erfindung Mittel zur Durchführung dieses Verfahrens, die dadurch gekennzeichnet sind, daß sie einen wasserunlöslichen Träger enthalten, an den die Antikörper gegen das zu bestimmende Enzym adsorptiv gebunden sind. Als wasserunlösliche Träger werden vorzugsweise Kunststoffgefäße verwendet, insbesondere Teströhrchen aus Polystyrol oder Polypropylen.

Voraussetzung für das erfindungsgemäße Verfahren ist die Erzeugung von Antiseren, die Enzyme bzw. Isoenzyme spezifisch binden können. Diese Bindung muß ausreichend stabil sein, und die effektiv gemessene enzymatische Aktivität der gebundenen Komponente darf nicht oder nur in einem reproduzierbaren Maße gehemmt werden. Antiseren oder gereinigte Immungloboline aus solchen Antiseren in geeigneten Pufferlösungen werden adsorptiv an die Oberfläche von wasserunlöslichen Trägern wie Kunststoffgefäße aus Polystyrol, Polypropylen usw. gebunden. Ungebundene Antikörper aus dem Antiserum oder der Immunglobulinlösung werden durch Auswaschen der Gefäße entfernt. Zum Nachweis der Enzyme bzw. Isoenzyme wird die biologische Probe eine definierte Zeit in den Antikörper-beschichteten Gefäßen inkubiert. Dabei wird ein Anteil des zu bestimmenden Enzyms bzw. Isoenzyms, der der Probenkonzentration proportional ist, an der Antikörperschicht gebunden. Durch die Verwendung von Antikörpern, die fest binden, gelingt eine Anreicherung der Enzymmoleküle in der Antikörperschicht. Dies ist ein besonderer Vorteil z. B. beim Einsatz großer Volumina von Proben mit niedriger Konzentration des zu bestimmenden Enzyms bzw. Isoenzyms.

Die Restflüssigkeit der Probe wird anschließend entfernt und die Gefäße werden mehrmals mit einer üblichen Spüllösung aus detergentienhaltiger Pufferlösung gewaschen. Durch diesen Schritt werden Störfaktoren für den Enzymtest, die eventuell in der Probe vorhanden sind (Salze, Pharmaka, störende Enzyme), entfernt. Zum Nachweis der gebundenen Enzymmoleküle werden definierte Volumina von Lösungen mit Testreagenzien für den Enzymtest (enthaltend Substrate, Puffer, usw.) in die Gefäße gegeben. Die Extinktion der entstehenden Reaktionsprodukte der Enzymreaktion wird entweder direkt im Photometer registriert (kinetischer Enzymtest) oder nach definierter Reaktionszeit gemessen (Zwei-Punkt-Bestimmung). Um Absolutwerte für die Aktivität des zu bestimmenden Enzyms bzw. Isoenzyms in der Probe zu erhalten, wird eine Eichkurve mit Proben einer Kontroll-Lösung, deren Enzymaktivität vorgegeben ist, aufgestellt. Diese Proben wurden in gleicher Weise wie die Analysenproben behandelt. Man erhält dann eine Referenzkurve, die eine quantitative Bestimmung der Enzymaktivität in der Probe erlaubt.

Das erfindungsgemäße Verfahren ist überraschend einfach, spezifisch und empfindlich. Es vereinigt die Vorteile des Konzentrierungseffekts und der Elimination von Störfaktoren, weil die Enzymaktivität erst nach Entfernen der Probelösung bestimmt wird. Im Vergleich zu anderen immunologischen Methoden erfolgt eine direkte Messung, d. h. ein Reaktionsschritt mit markierten Antikörpern entfällt. Damit entfällt auch die Notwendigkeit, ein Kupplungsprodukt herzustellen, mit allen damit verbundenen Nachteilen. Als ein weiterer wesentlicher Faktor ist die Schnelligkeit hervorzuheben, mit der das erfindungsgemäße Verfahren durchgeführt werden kann.

### Beispiel 1

Bestimmung des Placenta-Isoenzyms der alkalischen Phosphatase

#### a) Herstellung des Isoenzym-spezifischen Antiserums

Alkalische Phosphatase aus menschlicher Placenta wurde gereinigt, bis das Enzym frei von anderen Isoenzymen der alkalischen Phosphatase war und andere Proteine in der Elektrophorese auf Polyacrylamidgel nicht mehr nachweisbar waren. Das Isoenzym wurde gegen 0,15 M Kochsalzlösung dialysiert, durch Zentrifugation von Aggregaten befreit und sterilfiltriert. Der Proteingehalt der Lösung wurde mit einer 0,15 M Kochsalzlösung auf 2 mg/ml eingestellt. 1 ml dieser Lösung wurde mit 1 ml komplettem Freund'schen Adjuvans emulgiert. Diese Emulsion wurde Schafen bzw. Ziegen an 3 bis 4 Stellen i. m. in die Hinterläufe injiziert. Gleiche Injektionen erfolgten noch zweimal im Abstand von je drei Wochen. 21 Tage nach der letzten Injektion wurde Blut entnommen. Weitere Injektionen erfolgten im Abstand von etwa 12 Wochen nach der letzten Blutentnahme. Blut wurde jeweils drei Wochen nach der Boosterinjektion entnommen. Aus dem Blut wurde Serum nach bekannten hämatologischen Verfahren gewonnen.

#### b) Isolierung der Immunglobulinfraktion aus dem Antiserum

Die IgG-Fraktion wurde aus den Antiseren durch Kombination von Ammonsulfatfällung, Dialyse gegen 0,071 M Acetatpuffer vom pH 5 und Ionenaustauscherchromatographie über DEAE-Sephadex bei pH 5 nach einem in Scand. J. Immunol. 2, Suppl. 1, 161—164 (1973) beschriebenen Verfahren isoliert.

#### c) Beschichtung von Reaktionsgefäßen mit Antikörpern

Als Reaktionsgefäße für die Beschichtung mit Antikörpern wurden Reagenzröhrchen aus Polystyrol 12 x 55 mm verwendet. Je ml einer IgG-Lösung mit einer Konzentration von 10 µg/ml in 0,15 M Kochsalzlösung mit 0,02% Natriumazid wurde in die Reagenzröhrchen gegeben und die verschlossenen Gefäße wurden 18 Stunden bei 4°C inkubiert. Anschließend wurde die Flüssigkeit durch Dekantieren oder Absaugen entfernt; die Gefäße wurden mehrmals mit Spüllösung (0,05% eines Detergenz in 0,15 M Kochsalzlösung mit 0,02% Natriumazid) gewaschen und an der Luft getrocknet.

#### d) Bestimmung des Placenta-Isoenzyms in biologischen Proben

Als biologische Probe wurde hitzeinaktiviertes Serum von gesunden Männern eingesetzt, dem Placenta-Isoenzym der alkalischen Phosphatase mit bekannter Enzymaktivität zugemischt wurde. Für die Versuche zur Wiederfindung von AP-Placenta wurden Gemische aus kommerziell erhältlichen Kontrollseren mit AP-Placenta als alleiniger AP-Aktivität und dem hitzeinaktivierten Humanserum verwendet. In die mit Antikörpern beschichteten Gefäße wurden Proben mit 0,1 ml des AP-Placenta-haltigen Serums einpipettiert.

Die Röhrchen wurden auf 0,5 ml aufgefüllt mit 10 mM Tris/HCl-Puffer pH 7,5, der zusätzlich 2 mM Magnesiumchlorid und 0,025 mM Zinkchlorid enthält. Die Gefäße wurden verschlossen und eine Stunde bei 37°C sowie ca. 16 Stunden bei 4°C unter sanftem Schütteln inkubiert. Anschließend wurden die Gefäße dreimal mit Tris/HCl-Puffer (10 mM, pH 7,5 mit 2 mM Magnesiumchlorid und 0,025 mM Zinkchlorid) gewaschen und kurz an der Luft getrocknet. Dann wurden pro Gefäß 1,0 ml Puffer-Substrat-Lösung für die Bestimmung der Aktivität der alkalischen Phosphatase zugegeben und 10 Min. bei Zimmertemperatur inkubiert. Sofort danach wurde die Extinktion der Probe im Photometer bei 405 nm in einer Küvette gemessen. Als Blindwert wurde eine gleichbehandelte Puffer-Substratlösung verwendet. Die Auswertung erfolgte anhand einer Eichkurve, wobei zwischen der eingesetzten bekannten Aktivität der AP-Placenta in der Probe (U/l) und der gemessenen Extinktionsänderung pro Zeiteinheit (10 Min.) eine strenge Korrelation bestand (Korr. Koeff. r=0,998). Aus der gebundenen Aktivität wurde errechnet, daß etwa 70 bis 80% der AP-Placenta-Aktivität an den Gefäßen in gebundener Form wiedergefunden wurde.

Als Puffer-Substrat-Lösung wurde eine Lösung verwendet, die folgende Konzentrationen im Test hat: 10 mM p-Nitrophenylphosphat, 0,5 mM Magnesiumchlorid, 1,0 mM Diäthanolamin-HCl-Puffer pH 9,8.

### e) Kinetische Bestimmung des gebundenen Placenta-Isoenzyms der alkalischen Phosphatase

Es wurden Proben von 0,1 ml Humanseren mit ca. 80 U/l AP-Placenta-Aktivität verwendet. Die Proben wurden in Antikörper-beschichteten Viereck-Küvetten aus Polystyrol inkubiert.

Diese Gefäße wurden inkubiert und danach gewaschen, wie unter 1d beschrieben. Zum Nachweis der gebundenen AP-Placenta wurde die Puffer-Substrat-Lösung in diese Gefäße gegeben und die Extinktionsänderung durch das entstehende Reaktionsprodukt in den Gefäßen direkt im Photometer mit Schreiberzusatz registriert: Die Reaktion läuft ca. 2 Min. linear.

### f) Bestimmung des Placenta-Isoenzyms der alkalischen Phosphatase in Proben mit zugesetzten Enzyminhibitoren

0,05 ml Humanserum mit einer AP-Placenta-Aktivität von ca. 140 U/l wurden mit jeweils 0,5 ml 10 mM Tris/HCl-Puffer vom pH 7,5 gemischt, der folgende Zusätze enthielt: 2 mM Magnesiumchlorid und 0,025 mM Zinkchlorid (A), Puffer wie in (A) mit 5 mM L-Phe-Gly-Gly (B), Puffer wie in (A) mit 5 mM L-Leu-Gly-Gly (C), Puffer wie in (A) mit 5 mM L-Phenylalanin (D). Die Aktivität dieser Proben wurde analog Beispiel 1d bestimmt. Alle Ansätze ergaben Werte für die Antikörper-gebundene AP-Placenta (gemessen als $\Delta$OD/10 Min.), die mit dem Vergleichswert aus (A) innerhalb der Fehlergrenze der Methode identisch waren. Die Gegenwart der Hemmstoffe für AP-Placenta in der Probe störte die exakte Bestimmung mit Antikörper-beschichteten Gefäßen nicht.

### Beispiel 2

### Bestimmung des Isoenzyms der alkalischen Phosphatase aus Leber bzw. Knochen (AP-Leber/Knochen)

Das Isoenzym wurde aus Humanleber isoliert und gereinigt, bis das Protein frei von anderen Isoenzymen der alkalischen Phosphatase war. Die Immunisation erfolgte analog Beispiel 1a. Das Antiserum wurde mit Glutaraldehyd-vernetztem Humanserum absorbiert. Die Isolierung der Immunglobulinfraktion aus dem absorbierten Antiserum erfolgte analog Beispiel 1b und die Beschichtung von Reaktionsgefäßen mit den Antikörpern analog Beispiel 1c. Zur Bestimmung der Aktivität von AP-Leber/Knochen wurde als Probe ein hitzeinaktiviertes Humanserum mit zugesetzter alkalischer Phosphatase aus Humanleber verwendet. Die Totalaktivität der alkalischen Phosphatase (identisch mit der Aktivität der AP-Leber/Knochen) in der Probe wurde vor der Bestimmung ermittelt.

In die Reaktionsgefäße, die mit Antikörpern gegen AP-Leber/Knochen beschichtet waren, wurden 0,1 ml Probe und 0,4 ml 10 mM Tris/HCl-Puffer pH 7,5 mit 2 mM Magnesiumchlorid und 0,025 mM Zinkchlorid pipettiert. Die weitere Behandlung wurde analog Beispiel 1d durchgeführt. Die Auswertung erfolgte anhand einer Eichkurve, wobei zwischen der eingesetzten bekannten Aktivität der AP-Leber/Knochen in der Serumprobe (U/l) und der gemessenen Extinktionsänderung pro Zeiteinheit (10 Min.) eine strenge Korrelation bestand (Korr. Koeff. r = 0,997).

### Beispiel 3

### Bestimmung der Dünndarm-AP im Serum, Plasma oder Harn

Das Dünndarm-Isoenzym der alkalischen Phosphatase wurde aus menschlichem Dünndarm isoliert und gereinigt, bis das Protein frei von anderen Isoenzymen der alkalischen Phosphatase war. Die Immunisation erfolgte analog Beispiel 1a. Das Antiserum wurde mit Glutaraldehyd-vernetztem Humanserum absorbiert. Aus dem Antiserum nach der Absorption wurde die Immunglobulin G-Fraktion gereinigt (Meth. Enzymol. XXXIV, S. 725, Academic Press, New York 1974). Zur Beschichtung wurden Polystyrol-Röhrchen (Inhalt 1 ml) 20 Stunden mit einer Lösung von Anti-Dünndarm-AP-IgG (10 µg/ml) in 30 mM Barbitalpuffer pH 8,6 inkubiert, zweimal mit je 1 ml Phosphat-gepufferter physiologischer Kochsalzlösung gewaschen, mit physiologischer Kochsalzlösung gefüllt und bei 4°C aufbewahrt. Von Serum oder Plasma wurden 0,1 ml, von Harn bis zu 1 ml Probenvolumen eingesetzt. Die Röhrchen wurden dann auf 1 ml aufgefüllt mit 10 mM Tris/HCl-Puffer pH 7,5 mit 0,15 M Natriumchlorid, 2 mM Magnesiumchlorid, 0,025 mM Zinkchlorid, 0,2% Rinderalbumin und 3% Polyäthylenglycol 6000 und danach 24 Stunden bei 4°C inkubiert. Der Überstand wurde abgesaugt und jedes Röhrchen dreimal mit Kochsalzlösung, die zusätzlich 3% Polyäthylenglycol 6000 enthielt, gewaschen. Die gewaschenen Röhrchen wurden mit 0,95 ml einer Testlösung für alkalische Phosphatase (1 M Diäthanolamin/HCl-Puffer pH 9,8 mit 10 mM p-Nitrophenylphosphat und 0,5 mM Magnesiumchlorid) gefüllt. Die Reaktion wurde nach 60 Min. durch Zugabe von 50 µl einer Lösung von 5 N Natronlauge und 10 mM Äthylendinitrilotetraessigsäure-Dinatriumsalz gestoppt. Als Eichantigen wurde reine, aus menschlichem Dünndarm isolierte alkalische Phosphatase eingesetzt. Die Auswertung erfolgte analog Beispiel 1d. Dabei ergab sich für die Dünndarm-AP eine untere Nachweisgrenze von 0,05 U/l (entsprechend 25 ng/l) bei einem Probevolumen von 1 ml bzw. 0,5 U/l (entsprechend 250 ng/l) bei einem Probevolumen von 0,1 ml.

### Beispiel 4

#### Bestimmung des Prostata-Isoenzyms von menschlicher saurer Phosphatase

Das Isoenzym der sauren Phosphatase wurde aus menschlichem Prostatagewebe extrahiert und mittels fraktionierter Ammoniumsulfatfällung vorgereinigt. Die anschließende Isolierung des reinen Proteins erfolgte nach Prep. Biochem. 8, 73—89 (1978). Die spezifische Aktivität des reinen Isoenzyms betrug ca. 385 U/mg Protein. Zur Gewinnung von Antikörpern wurden Kaninchen immunisiert. 1 mg des reinen Proteins in 1 ml Lösung wurde mit 1 ml kompletten Freund'schen Adjuvans emulgiert und dem Tier subcutan am Rücken injiziert. Eine zweite Injektion erfolgte 14 Tage später. Nach 4 Wochen wurde die halbe Menge der Emulsion intramuskulär injiziert. 7—10 Tage später wurde aus der Ohrarterie Blut entnommen. Aus dem Antiserum wurde die IgG-Fraktion mittels Affinitätschromatographie nach FEBS Letters 28, 31 (1972) isoliert.

Zur Beschichtung wurden Polystyrolröhrchen (Inhalt 1 ml) 20 Stunden mit einer Lösung der IgG-Fraktion aus dem Antiserum gegen das Prostata-Isoenzym (5 µg IgG/ml in 30 mM Barbitalpuffer pH 8,6) inkubiert, zweimal mit je 1 ml Phosphat-gepufferter physiologischer Kochsalzlösung gewaschen, mit Kochsalzlösung gefüllt und bei 4° C aufbewahrt. Für den Test wurde Humanserum oder -plasma (max. 1 ml) 24 Stunden bei 4° C in Röhrchen inkubiert, der Überstand abgesaugt und jedes Röhrchen dreimal mit Phosphat-gepufferter physiologischer Kochsalzlösung gewaschen, die 3% Polyäthylenglykol 6000 und 0,1% Detergenz enthielt. Zum Nachweis des gebundenen Enzyms wurden 0,95 ml einer Testlösung für saure Phosphatase (50 mM Citratpuffer pH 4,8 mit 5,5 mM p-Nitrophenylphosphat) in die gewaschenen Röhrchen gefüllt. Nach 60 Min. wurde die Enzymreaktion durch Zusatz von 50 µl 3 N Natronlauge gestoppt und die Extinktionszunahme bei 405 nm gegenüber einem Blindwert bestimmt. Der Blindwert wurde mit Hilfe eines gleichbehandelten Röhrchens ermittelt, das mit physiologischer Kochsalzlösung und 3% Polyäthylenglykol 6000 anstelle von Serum 24 Stunden bei 4° C inkubiert wurde.

Es wurde eine Eichkurve aufgestellt, bei der die Aktivität der eingesetzten sauren Phosphatase mit der im Röhrchen meßbaren Restaktivität korreliert. Die Eichkurve wurde durch Zugabe einer definierten Aktivität des Prostata-Isoenzyms der sauren Phosphatase zu einem Gemisch von Frauenseren bestimmt. Die Blindaktivität des Serums wurde jeweils von den gemessenen Werten abgezogen. Die Nachweisgrenze lag bei 0,05 U/l (entsprechend 120 ng/l); die Empfindlichkeit ist somit mehr als doppelt so gut wie die eines kommerziellen Radioimmunoassays (250 ng/l).

### Patentansprüche

1. Verfahren zur quantitativen immunologischen Bestimmung von Enzymen in Flüssigkeiten, dadurch gekennzeichnet, daß man die an einem wasserunlöslichen Träger adsorbierten Antikörper gegen das zu bestimmende Enzym mit der dieses Enzym enthaltenden Probelösung inkubiert, wobei das Enzym im Komplex mit den Antikörpern einen reproduzierbaren Anteil seiner Aktivität behält, die Probelösung entfernt, den mit Enzym beladenen Antikörper-Träger wäscht und die Aktivität des an die Antikörper gebundenen Enzyms bestimmt.

2. Verfahren nach Anspruch 1, dadurch gekennzeichnet, daß Isoenzyme der sauren bzw. alkalischen Phosphatase in Körperflüssigkeiten bestimmt werden.

3. Verfahren nach Anspruch 1, dadurch gekennzeichnet, daß das Prostata-Isoenzym der sauren Phosphatase in Körperflüssigkeiten bestimmt wird.

4. Mittel zur Durchführung des Verfahrens nach den Ansprüchen 1 bis 3, dadurch gekennzeichnet, daß es einen wasserunlöslichen Träger enthält, an den die Antikörper gegen das zu bestimmende Enzym adsorptiv gebunden sind.

5. Mittel nach Anspruch 4, dadurch gekennzeichnet, daß der wasserunlösliche Träger ein Kunststoffgefäß ist.

6. Mittel nach den Ansprüchen 4 und 5, dadurch gekennzeichnet, daß der wasserunlösliche Träger ein Teströhrchen aus Polystyrol oder Polypropylen ist.

### Claims

1. Process for the quantitative immunological determination of enzymes in liquids, characterised in that antibodies against the enzyme to be determined, which are adsorbed on a water-insoluble carrier, are incubated with the sample solution containing this enzyme, the enzyme retaining a reproducible proportion of its activity in the complex with the antibodies, the sample solution is removed, the antibody carrier, which is charged with enzyme, is washed and the activity of the enzyme bonded to the antibodies is determined.

2. Process according to Claim 1, characterised in that isoenzymes of the acid or alkaline phosphatase in body fluids are determined.

3. Process according to Claim 1, characterised in that the prostate isoenzyme of acid phosphatase in body fluids is determined.

4. Agent for carrying out the process according to one of Claims 1 to 3, characterised in that it contains a water-insoluble carrier to which the antibodies against the enzyme to be determined are bonded by adsorption.

5. Agent according to Claim 4, characterised in that the water-insoluble carrier is a plastic vessel.

6. Agent according to Claims 4 and 5, characterised in that the water-insoluble carrier is a polystyrene or polypropylene test tube.

## Revendications

1. Procédé pour la détermination immunologique quantitative d'enzymes dans des liquides, caractérisé en ce que l'on soumet les anticorps contre l'enzyme à déterminer, adsorbés sur un support insoluble dans l'eau, à incubation avec la solution échantillon contenant cette enzyme, l'enzyme conservant dans le complexe avec les anticorps une proportion reproductible de son activité, on élimine la solution échantillon, on lave le support des anticorps chargé d'enzyme et on détermine l'activité de l'enzyme fixée sur les anticorps.

2. Procédé selon la rev. 1, caractérisé en ce que l'on détermine les isoenzymes de la phosphatase acide ou alcaline dans des liquides corporels.

3. Procédé selon la rev. 1, caractérisé en ce que l'on détermine l'isoenzyme prostatique de la phosphatase acide dans des liquides corporels.

4. Réactif pour la mise en œuvre du procédé selon les rev. 1 à 3, caractérisé en ce qu'il contient un support insoluble dans l'eau sur lequel on a fixé par adsorption les anticorps contre l'enzyme à déterminer.

5. Réactif selon la rev. 4, caractérisé en ce que le support insoluble dans l'eau est un récipient de résine synthétique.

6. Réactif selon les rev. 4 et 5, caractérisé en ce que le support insoluble dans l'eau est un petit tube à essais en polystyrène ou en polypropylène.